# EUROPEAN PATENT APPLICATION

(11) **EP 1 760 056 A1**
(43) Date of publication of application: **07.03.2007**
(21) Application number: 05017898.7
(22) Date of filing: 17.08.2005
(51) Int. Cl.: C07B 59/00, C07C 269/06, C07C 271/22, A61K 51/04

(54) **A convenient method for the preparation of no-carrier-added O-(2-[18F]fluoroethyl)-L-Tyrosine)**

(62) Divisional of application: 05023701.5
(71) Applicant: Institute of Nuclear Energy Research, Lungtan Taoyuan, Taiwan 325 (TW)
(72) Inventor: Wang, Hsin Er Inst. of Nucl. Energy Res., Taoyuan Country 32546 Taiwan (TW); Wu, Shih Yan Inst. of Nucl. Energy Res., Taoyuan Country 32546 Taiwan (TW); Lin, Wuu Jyh Inst. of Nucl. Energy Res., Taoyuan Country 32546 Taiwan (TW); Chen, Jenn Tzong Inst. of Nucl. Energy Res., Taoyuan Country 32546 Taiwan (TW); Lo, Ai Ren Inst. of Nucl. Energy Res., Taoyuan Country 32546 Taiwan (TW); Lee, Ming Hsin Inst. of Nucl. Energy Res., Taoyuan Country 32546 Taiwan (TW); Chang, Mao Hsiung Inst. of Nucl. Energy Res., Taoyuan Country 32546 Taiwan (TW)
(74) Representative: Hager, Thomas Johannes

(57) **Abstract**

This is a novel method for production of no-carrier-added *O*-(2-[¹⁸F]fluoroethyl)-L-Tyrosine which has been proved a suitable PET (position emission tomography) probe for tumor diagnosis imaging.

The preparation of the title compound starts from precursors with the chemical structures as in Formula 1, wherein R¹ is a protective group for the carboxyl functional group, R² is a protective group for the amino group, and R³ acts as a leaving group. R¹ represents an arylalkyl group, R² represents a carboxyl group, and R³ represents a p-tosyloxy, methane sulfonyloxy or trifluoromethane sulfonyloxy or bromine. The final purification of the product is using a separation column, which is very convenient for automated synthesis. The invention uses the precursor with the chemical structures as in Formula 1. 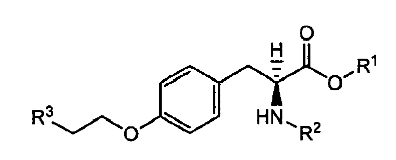 Formula 1 : Synthesis precursors for *O*-(2-C¹⁸F]fluoroethyl)-L-Tyrosine.

## Description

### BACKGROUND OF THE INVENTION

¹⁸F-labeled *O*-(2-[¹⁸F]fluoroethyl)-L-Tyrosine is an amino acid tracer, it has been proved as a suitable PET (position emission tomography) probe for tumor diagnosis imaging. Today, 2-[¹⁸F]Fluoro-2-Deoxy-D-glucose( [¹⁸F]FDG) has been widely used as a PET agent for evaluate tumor diagnosis and treatment through biochemical pathway of glucose metabolism. Increase brain background radiation due to high absorption of [¹⁸F]FDG in the brain cause diagnosis difficulty of brain tumor; High [¹⁸F]FDG absorption of inflammation site also cause misjudgment Brain tumor cells also have high concentration of L-[methyl-¹¹C] methionine, but free of diagnosis difficulty and misjudgment as described above; nevertheless, the half-life of L-[methyl-¹¹C] methionine is twenty minutes, therefore, attenuation rapidly during delivery with increasing cost even use cyclotron.

The absorption of ¹⁸F-labeled *O*-(2-[¹⁸F]fluoroethyl)-L-Tyrosine through biochemical pathway of amino acid metabolism almost the same as ¹¹C-labeled L-[methyl-"C]methionine, the absorption by brain tumor is much lower than [¹⁸F]FDG and also by inflammation site. Half-life of both [¹⁸F]FET and [¹⁸F]FDG are 109 minutes, this will facilitate the delivery between hospitals, and commercialization is possible because of its cost is lower than "C-labeled L-[methyl-¹¹C]methionine.

The preparation of *O*-(2-[¹⁸F]fluoroethyl)-L-Tyrosine was developed by Wester et al.(J. Nucl. Med. 1999;40:205-212) and Hamacher et al. (Appl. Radiat. Isot. 2002;57:853-856). However, it is inconvenient by using high performance liquid chromatography (HPLC) for purified product, it is not only cumbersome but also difficult automation, since during HPLC purification process operator must switch valve from waste collecting bottle to product bottle in order to collect purified *O*-(2-[¹⁸F]fluoroethyl)-L-Tyrosine and switch valve back to waste bottle after complete the collection of purified product in order to maintain high purified *O*-(2-[¹⁸F]fluoroethyl)-L-Tyrosine. This procedure is impotent, since bad control of valve will cause loss or impurity product and manufacturer must face with problems of unqualified product and increasing cost of manufacturing equipment.

### Cited references

1. H.J.Wester, M.Herz, W.Weber, P.Heiss, R.Senekowitsch-Schmidtke, M.Schwaiger and GStöcklin, Synthesis and radiopharmacology of O-(2-[18F]fluoroethyl)-L-Tyrosine for tumor imaging. J. Nucl. Med. 40, 205-212 (1999).
2. K.Hamacher and H.H.Coenen, Efficient routine production of the 18F-labelled amino acid O-(2-[18F]fluoroethyl)-L-Tyrosine, Appl. Radiat. Isot. 57, 853-856 (2002).

### SUMMARY OF THE INVENTION

The object of this invention provides a synthetic method of novel t-BOC-(*O*-tosyloxyethyl)-L-Tyr-OBzl (as Formula 1), which is a precursor of *O*-(2-[¹⁸F]fluoroethyl)-L-Tyrosine.

For the synthesis of *O*-(2-[¹⁸F]fluoroethyl)-L-Tyrosine, the initial labeled-compound (that is precursor) is prepared above all. This synthesis procedure is carried out in chemistry lab instead of in radiation control area because of no radiation.

### Precursor of O-(2-[¹⁸F]fluoroethyl)-L-Tyrosine

Synthesis of *O*-(2-[¹⁸F]fluoroethyl)-L-Tyrosine : (1) Prepare ethylene glycol-1,2-ditosylate. React ethylene glycol and toluenesulfonyl chloride in pyridine solution at low temperature for two to three days, warming the solution, solidify the product and purify by re-crystallization to obtain ethylene glycol-1,2-ditosylate. (2) Prepare t-Boc-(*O*-tosyloxyethyl)-L-Tyr-Obzl. Add t-Boc-L-Tyr-OBzl in acrylonitrile solution contains ethylene glycol-1,2-ditosylate and potassium carbonate, heat to 90°C in stirring for react four hours, remove solvent after complete the reaction, then extract with chloroform to obtain solid residue, purify dissolved residue by column chromatography and obtain pure t-Boc-(*O*-tosyloxyethyl)-L-Tyr-OBzl product.

Another object of the invention provides a synthetic method of a novel *O*-(2-[¹⁸F]fluoroethyl)-L-Tyrosine. It must be carry out in radiation control room or area when use this precursor as an initial labeling substance.

### Synthetic method of O-(2-[¹⁸F]fluoroethyl)-L-Tyrosine comprises:

(1) Use Kryptofix 2.2.2 as a catalyst. Perform substitution reaction of t-Boc-(*O*-tosyloxyethyl)-L-Tyr-OBzl with ¹⁸F ion in acrylonitrile solution under Kryptofix 2.2.2 catalysis and yield t-Boc-(*O*-[¹⁸F]fluoroethyl)-L-Tyr-OBzl , dry after purify by column chromatography, add 1N HCl after resultant resolve in alcohol, hydrolysis at temperature of 100°C, obtain isotonic solution by neutralize with 1N NaOH, then obtain final product after filter by 0.22 µm bacteria-free filtering film.
(2) Use Tetrabutylammonium bicarbonate(TBAHCO₃) as a catalyst. Perform substitution reaction of t-Boc-(*O*-tosyloxyethyl)-L-Tyr-OBzl with ¹⁸F ion in acrylonitrile solution under TBAHCO₃ catalysis and yield t-Boc-(*O*-[¹⁸F]fluoroethyl)-L-Tyr-OBzl, dry after purify by column chromatography, add 1N HCl after resultant resolve in alcohol, hydrolysis at temperature of 100°C, obtain isotonic solution by neutralize with 1N NaOH , then obtain final product after filter by 0.22 µm bacteria-free filtering film.
   Precursor used as an initial substance in the invention utilize resin and silica gel column for purification, the smooth synthetic procedure similar the production of [¹⁸F]FDG, therefore, automation is applicable. It is not necessary use high performance liquid chromatography (HPLC) for purification; the cost of synthetic equipment is lower as well. Experience technician of [¹⁸F]FDG synthesis in PET-CT center can operate this equipment easily.
   The advantage of this invention in nuclear medicine includes: (1) Use ¹⁸F nuclide with 109 minutes half-life as radioactive tracer; it increases reliability of supply for clinical use of PET-CT center in compare to ¹¹C and the distribution to other medical center is also possible. (2) Utilize resin and silica gel column for purification; the smooth synthetic procedure suitable for automatic production will enhance the convenience and repeatability of synthesis and decrease it production time as well as labor cost. (3) Save manufacturing cost and simplify flow processes without using HPLC equipment, that is, raise the success probability of synthesis and qualification ratio of quality control.

### BRIEF DESCRIPTION OF THE DRAWINGS

Figure 1 is the L-[¹⁸F]FET intake of F98 glioma cells at different time points;
Figure 2 is the accumulated radioactivity in organs at different time points after injected L-[¹⁸F]FET into tumor-bearing rat;
Figure 3 is the microPET imaging of left-brain F98 rat glioma implanted Fischer344 rat after inject L-[¹⁸F]FET at 30, 60, 90 and 120 minutes respectively; upper row represent the coronal scan and lower row represent the cross section scan; and
Figure 4 is the PET imaging of left-brain F98 rat glioma implanted Fischer344 rat after inject L-[¹⁸F]FET at 30, 60, 90 and 120 minutes respectively; upper row represent the coronal scan and lower row represent the cross section scan.

### DETAILED DESCRIPTION OF THE INVENTION

Embodiments of the invention will be described as follows:

### Example 1. The synthetic method of ¹⁸F-labeled precursor t-BOC-(O-tosyloxyethyl)-L-Tyr-OBzl

### 1. Preparation of ethylene glycol-1,2-ditosylate

(1) Add 17g of toluenesulfonyl chloride (TsCl)(F.W.=190.65 , 0.089 mol) into conical flask (A) with 20 ml of pyridine.
(2) Add 1.1 ml of ethylene glycol (F.W.=62.07 , 0.018 mol) into conical flask (B) with 30 ml of pyridine.
(3) Pour solution of conical flask (A) into conical flask (B) under the temperature of dry ice-acetone bath (-30°C approximately) and then put the flask under -18°C promptly for react two to three days.
(4) After complete reaction, pour the reactant in conical flask (B) into 500 ml beaker with ice water and cracked ice; white solid substance will appear after stirring.
(5) Add optimal 1N HCl into beaker above-mentioned and adjust pH to 6~7.
(6) Filter and collect white solid substance, then re-crystallize in mixture of methylene chloride and normal hexane to yield 80% of 5.33g ethylene glycol-1,2-ditosylate.

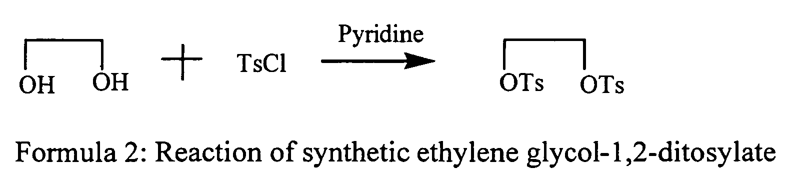

Following is the synthetic process of using t-BOC-L-Tyr-OBzl as a raw material (Formula 2): 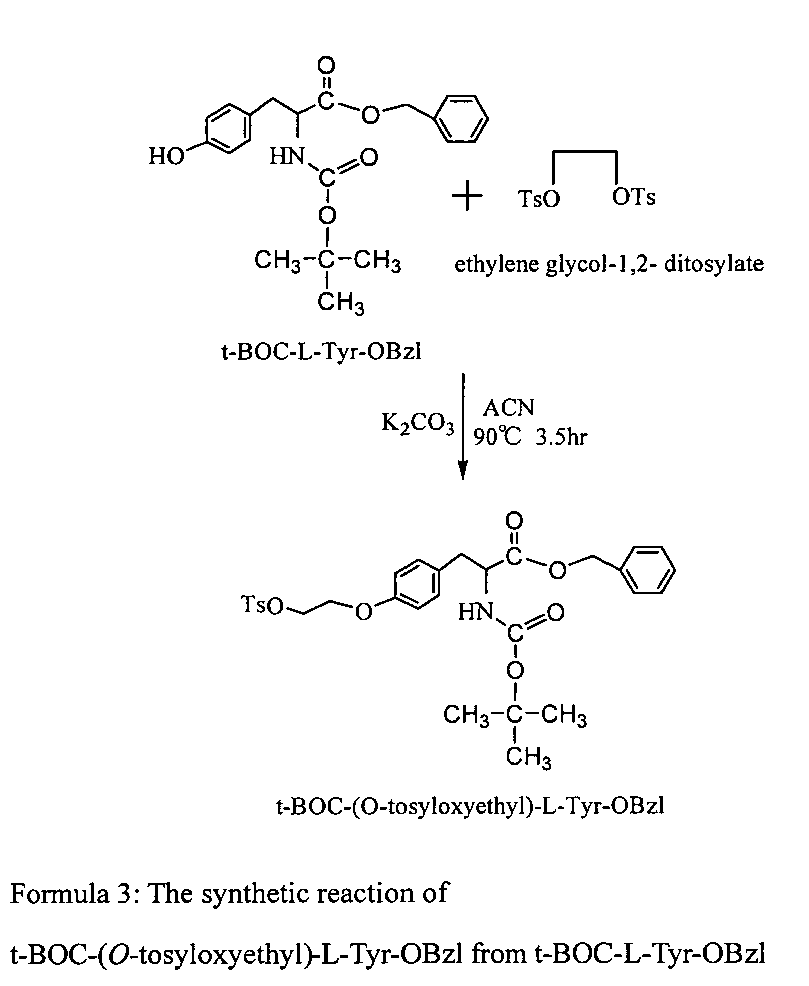
(1) Add 450mg of *N*-*tert*-butyloxycarbonyl- L-tyrosine benzylester (t-BOC-L-Tyr-OBzl) (F.W.=361, 1.24 mmol) into 50ml of round bottomed flask contains 20mg potassium carbonate and 1.384g ethylene glycol-1,2- ditosylate (F.W.=370.35 , 3.73 mmol); then add 25ml anhydrous acetonitrile at 90°C and stir for 3.5 hours.
(2) After complete the reaction, remove solvent with rotavapor and extract with chloroform (5ml x3). Harvest chloroform extract and remove solvent under negative pressure.
(3) Dissolve solid residue with minimum amount of methylene chloride, then perform silica gel chromatography (add 0.1% triethylamine in eluent) for purification. The initial condition of mobile phase is 100% CH₂Cl₂; after remove un-react ethylene glycol-1,2-ditosylate the condition of mobile phase change to CH₂Cl₂/CHCl₃=1/1, the crude product then eluted. Dry crude product under reduced pressure and obtain the solid crude product.
(4) Dissolve crude product with minimum volume solution of CH₂Cl₂:CHCl₃=8/2, then perform silica gel chromatography (add 0.1% triethylamine in eluent) for purification; the initial condition of mobile phase is CH₂Cl₂/CHCl₃=8/2(another 0.1% triethyl amine is added); light yellow oil-like substance (398 mg) of pure *N*-*tert*-butyloxycarbonyl-(*O*-tosyloxyethyl)-L-tyrosine benzylester (t-BOC-(*O*-tosyloxyethyl)-L-Tyr-OBzl) is eluted; re-crystallize in dichloromethane and n- hexane to yield 60.1% of white solid substance, melt point 85~86°C.
(5) Nuclear Magnetic Resonance (NMR): Dissolve 20mg of t-BOC-(*O*-tosyloxyethyl)-L-Tyr-OBzl in 0.6ml CDCl₃, then determine its ¹H-NMR spectrum (Formula 3). ¹H NMR (CDCl₃) δ 7.80 (d, 2H, J=8.4 Hz, Haryl), 7.31 (m, 7H, Haryl), 6.89 (d, 2H, J=8.4 Hz, Haryl), 6.62 (d, 2H, J=8.4 Hz, Haryl), 5.15 (d, 1H, 12.2 Hz, CH of benzyl), 5.08 (d, 1H, 12.2 Hz, CH of benzyl), 4.92 (d, 1H, J=8.0Hz, NH), 4.54 (m, 1H, CH), 4.33 (t, 2H, J=4.6 Hz, CH₂), 4.07 (t, 2H, J=4.6 Hz, CH₂), 2.99 (d, 2H, J=5.8 Hz, CH₂ of Tyr), 2.43 (s, 3H, CH₃ of toluene), 1.39 (s, 9H, CH₃ of t-BOC).
(6) Elemental analysis: The formula of t-BOC-(*O*-tosyloxyethyl)-L-Tyr-OBzl is C₃₀H₃₅NO₈S, the calculated value of elemental analysis is: C, 63.27; H, 6.15; N, 2.46. The actual value is: C, 63.34; H, 5.62; N, 2.33.

### Example 2. Use Kryptofix 2.2.2 as a catalyst to produce O-(2-[¹⁸F]fluoroethyl)-L-Tyrosine

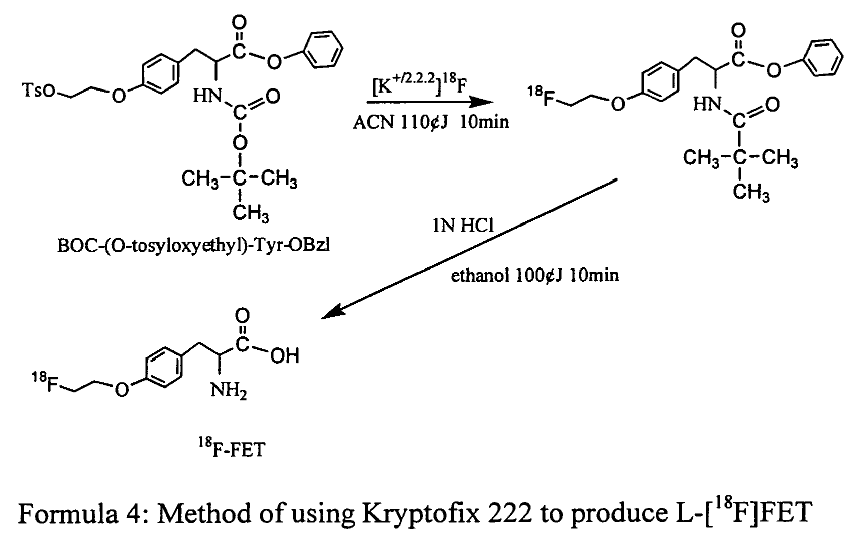

(1) Pour [¹⁸F]HF solution (0.5-1.3 ml , radioactivity 1-500 mCi) into 5ml pointed-base bottle with potassium carbonate (4.6 mg) and kryptofix 222 (26 mg); heat under 130°C, purge with nitrogen gas (200ml/min) and vacuum smoothly until the surface of liquid almost dry.
(2) Add slowly 3ml anhydrous acetonitrile in eight minutes while heat under 130°C; then purge with nitrogen gas (200ml/min) and vacuum smoothly so that azeotropic dry moisture and acetonitrile.
(3) Dissolve 5mg t-BOC-(O-tosyloxyethylhL-Tyr-OBzl in 0.8ml anhydrous acetonitrile, then add into above bottle at 110°C for 10mins reaction.
(4) After complete the reaction , insert evacuation needle while maintaining at 110°C and purge with nitrogen gas (200ml/min) until dry (take about 5 minutes to eliminate acetonitrile).
(5) Cold the reaction bottle to room temperature, adds 1.5ml CHCl₃ for dissolve reaction mixture and determine its radioactivity. Take a small fraction of solution for thin-layer chromatography (silica gel plate, developing agent CH₂Cl₂/CHCl₃=8/2). Pass above solution through pre-conditioned silica column while increase flow rate by weak hydrogen gas pressure; wash bottle with 1.5ml CHCl₃ before pass through the same silica column and the discarded effluent fluid is collected by waste bottle. Again, use 2.5 ml ether for silica column elution and collect effluent ether solution with capped test tube to obtain primary product have protective group. Take a small fraction of collected solution for thin-layer chromatography (silica gel plate, developing agent CH₂Cl₂/CHCl₃=8/2). Determine the radioactivity of collected solution, silica column and reaction bottle.
(6) Put capped test tube into 40°C water bath, purge with nitrogen (200ml/min) and aspirate slowly until dry. Add 0.3ml 1N hydrochloride after solids dissolve by 0.3ml ethanol and put into heating block (100°C) for ten-minute hydrolytic reaction.
(7) Take out bottle after complete reaction, neutralize with 0.35ml 1N sodium hydroxide solution and add additional 1.35ml pure water to constitute a total volume of 2ml isotonic solution, then cold to room temperature.
(8) Pass product through 0.22 µm bacteria-free filtering film and enter aseptic bottle as to obtain bacteria-free and carrier-free L-[¹⁸F]FET solution; determine radioactivity of filtered product (yield 30~40%, decay corrected) and analyze radiochemical purity (>90%) by thin-layer chromatography (reverse C 18 plate, acetonitrile /10 mM ammonium acetate=7/3).

### Example 3: Use tetrabutylammonium bicarbonate (TBA⁺HCO₃⁻) as a catalyst to produce O-(2-[¹⁸F]fluoroethyl)-L-Tyrosine

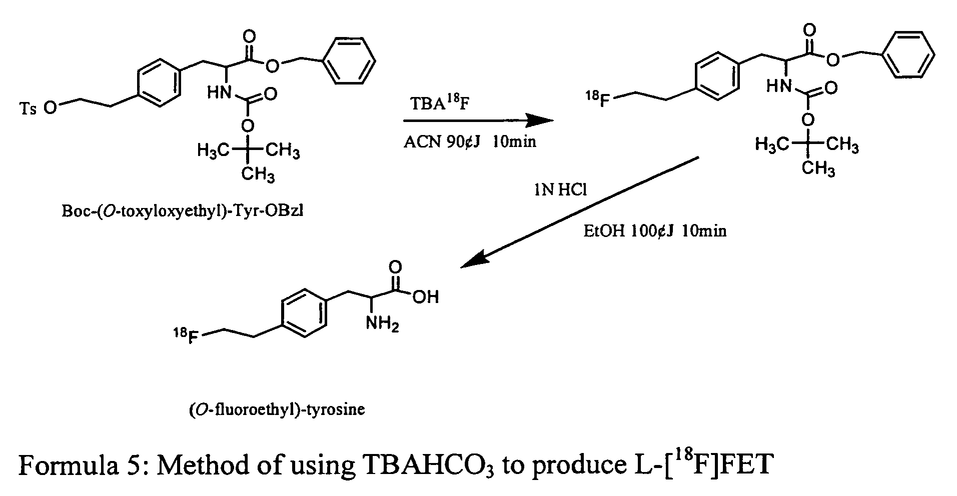

(1) Determine radioactivity of [¹⁸F]HF solution. Use peristaltic pump push [¹⁸F]HF solution through preconditioned QMA Sep-pak (1 ml/min), discard effluent fluid passed QMA (into [¹⁸O] water-returnable bottle). Determine radioactivity of effluent fluid and QMA Sep-pak.
(2) Elute QMA Sep-pak (1ml/min) with 0.8ml TBAHCO₃/ACN and collect effluent fluid of TBAHCO₃ into pointed-base bottle. Heat the bottle with heating block (100□), purge with nitrogen gas (200ml/min) and vacuum smoothly until the surface of liquid almost dry.
(3) Add 2ml of anhydrous acetonitrile slowly in eight minutes while heating at 100°C and purge with nitrogen gas (200ml/min). Vacuums smoothly so that azeotropic dry moisture and acetonitrile.
(4) Dissolve 5mg t-BOC-(*O*-tosyloxyethyl)-L-Tyr-OBzl in 0.8ml anhydrous acetonitrile, , then add into above bottle at 90°C for react 10 minutes.
(5) After complete the reaction, insert evacuation needle while maintaining at 90°C and purge with nitrogen gas (200ml/min) until dry (take about 5 minutes to eliminate acetonitrile).
(6) Cold the reaction bottle to room temperature. Adds 1.5ml CHCl₃ and shakes for dissolve reaction mixture before determine its radioactivity. Take a small fraction of solution for thin-layer chromatography (silica gel plate, developing agent CH₂Cl₂/CHCl₃=8/2). Pass above solution through pre-conditioned silica column while increase flow rate by weak hydrogen gas pressure; wash bottle with 1.5ml CHCl₃ before pass through the same silica column and the discarded effluent fluid is collected by waste bottle. Again, use 2.5 ml ether for silica column elution and collect effluent ether solution with capped test tube to obtain primary product have protective group. Take a small fraction of collected solution for thin-layer chromatography (silica gel plate, developing agent CH₂Cl₂/CHCl₃=8/2). Determine the radioactivity of collected solution, silica column and reaction bottle.
(7) Put capped test tube into 40°C water bath, purge with nitrogen (200ml/min) and aspirate slowly until dry. Add 0.3ml 1N hydrochloride after solids dissolve by 0.3ml ethanol and put into heating block (100°C) for ten-minute hydrolytic reaction.
(8) Take out bottle after complete reaction, neutralize with 0.35ml 1N sodium hydroxide solution and add additional 1.35ml pure water to constitute a total volume of 2ml isotonic solution, then cold to room temperature.
(9) Pass product through 0.22 µm bacteria-free filtering film and enter aseptic bottle as to obtain bacteria-free and carrier-free L-[¹⁸F]FET solution; determine radioactivity of filtered product (yield 40~50%, decay corrected) and analyze radiochemical purity (>90%) by thin-layer chromatography (reverse C18 plate, acetonitrile /10 mM ammonium acetate=7/3).

### Example 4: Evaluation of L-[¹⁸F]FET uptake by F98 glioma tumor cells

(1) In five six-well plates, put 1×10⁶ F98 glioma cells and 2 ml DMEM (1g glucose/1) into three wells.
(2) Total of five time points (2, 5, 10, 30 minute and 1hour) in this experiment and each time point use one six-well plate.
(3) Predetermined activity of L-[¹⁸F]FET or [¹⁸F]FDG (10 µCi/well) is added into each well during the experiment. Radioactivity uptake of cells divided by total added radioactivity can obtain its calculated uptake ratio.
(4) Formula 4 shows the result of L-[¹⁸F]FET intake by F98 glioma cells. F98 glioma cells intake L-[¹⁸F]FET rapidly and maximal accumulation will reach after 10 minutes (uptake ratio 0.47%). As cell cannot metabolize L-[¹⁸F]FET, therefore, the rate of L-[¹⁸F]FET transportation by the cells become balance after ten minutes when add L-[¹⁸F]FET; the accumulation of L-[¹⁸F]FET in glioma cells only slightly decrease after 1 hour (uptake ratio 0.41%).

### Example 5: Biological distribution of L-[¹⁸F]FET in F98 glioma cells of Fischer 344 rat

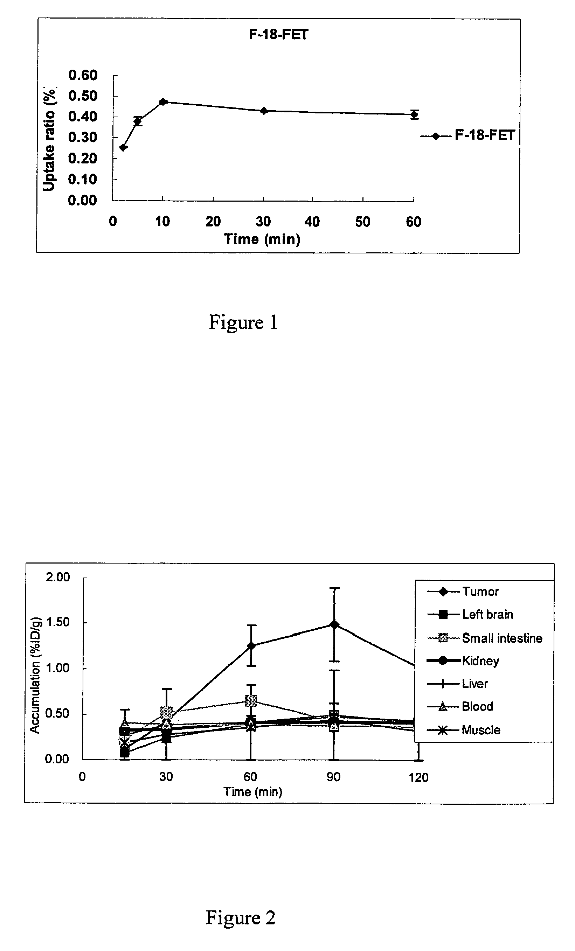

(1) Total of twenty Fischer 344 male rats will be assessed in vivo for L-[¹⁸F]FET biological distribution. Use the intersection of bregma and sagittal midlines as original point; so as to implant F98 glioma cells (1 × 10⁵cells /10 µl) at the place of 3mm left 5mm upward and 5mm depth. Biological distribution test will be carry out after 11~12 days of brain tumor implantation.
(2) Different volume of radioactive level is injected, injected volume of L-[¹⁸F]FET for each Fischer 344 rat is 200~300µl, that is approximately 200~250 µCi of activity.
(3) Inject L-[¹⁸F]FET via tail vein, and rats are sacrificed at 15, 30, 60, 90 and 120 minutes.
(4) Dissection of rat organ is right after sacrifice. Scissors off chest and abdomen fur alone the line from genital pore upward to jowl. Next, scissors off chest above xiphisternal transversely. Open ribs aside, take blood sample from venous sinus with 0.5 ml syringe. Withdraw the needle after depletion of blood and collect the extracted heart. Collect the lung lobes with forcep.
(5) Scissors off abdominal muscle from genital pore upward to the position below xiphisternal longitudinally. Then, scissors off the opening of upper and lower edges transversely, everts muscle layers to both sides and expose celiac organs. Search for bladder in lower abdomen and sequentially removes stomach, liver, spleen, small intestine, large intestine, kidney, and muscle.
(6) After turn over the rat, scissors off fur of shoulder and removes exposed subcutaneous tumor. Cut scalp upward at lower margin of cranium above foramen magnum and expose cranial bone. Then, cut cervical portion transversely at foramen magnum and expose spinal cord. Inserts foramen magnum with scissors' tip while attach the inner surface of cranium; such that scissors' tip can reach the frontal edge of cranial cavity in order to scissor off the center of cranium. Use scissors to open cranium aside and expose brain tissue. Alone skull base insert scissors deeply to the front edge of cranial cavity in order to remove left-brain, right-brain and brain tumor.
(7) Weight all organ samples; measure radioactivity with γ-counter and convert to %ID/g (% injection dose/g organ) of each organ for estimate the biological distribution of L-[¹⁸F]FET. From the observation of biological distribution find out that radiotracer accumulation of tissue may vary depend on the duration after intake L-[¹⁸F]FET; the radioactivity of each tissue is expressed by %ID/g (percent injected dose per gram of tissue); the experimental results are showed in table 1 and Figure 2. According to reports, L-[¹⁸F]FET can pass through blood brain barrier (BBB) and enter brain by way of L amino acid transporter system. This study result of L-[¹⁸F]FET biological distribution shows that maximum accumulation of L-[¹⁸F]FET in brain tumor and normal brain tissue is at 90 minutes, that is 1.49 and 0.48 %ID/g respectively. Tumor-to-normal brain ratio of L-[¹⁸F]FET at 15, 30, 60, 90 and 120 minutes are 1.54, 1.74, 3.16, 3.14 and 2.34 respectively. Besides brain tumor, rat pancreas also accumulate large amount of L-[¹⁸F]FET (0.98, 1.76, 2.55, 2.28 and 2.24 %ID/g respectively at 15, 30, 60, 90 and 120 minutes); the increasing radioactivity of L-[¹⁸F]FET in pancreas is because of pancreas require large amount of amino acid for synthesis various enzymes and hormones. Some papers reported that patient with tumor observes no significant accumulation of radioactivity in pancreas when use positron emission tomography (PET), the discrepant results were assumed that human and rat have different metabolic pathway. Furthermore, biological distribution study of L-[¹⁸F]FET indicated radioactivity accumulation in tumor cells (expressed by %ID/g) are much higher than other normal organs (left-brain, small intestine, kidney, liver, blood and muscle) except pancreas, this means L-[¹⁸F]FET can be a potential PET agent for substantially different site of tumor. Since L-[¹⁸F]FET is excrete via urine, therefore, high level of radioactivity will be found in urine sample.

**Table 1: Determination of accumulated radioactivity (%ID/g) in organs at different time points after injected L-[¹⁸F]FET into tumor-bearing rat**

| **Organ** | **15 min** | **30 min** | **60 min** | **90 min** | **120 min** |
|---|---|---|---|---|---|
| **Blood** | **0.41 ± 0.03** | **0.39 ± 0.03** | **0.38 ± 0.00** | **0.37 ± 0.03** | **0.36 ± 0.03** |
| **Lung** | **0.32 ± 0.05** | **0.43 ± 0.09** | **0.43 ± 0.07** | **0.45 ± 0.02** | **0.40 ± 0.06** |
| **Heart** | **0.30 ± 0.06** | **0.40 ± 0.07** | **0.43 ± 0.07** | **0.47 ± 0.02** | **0.43 ± 0.07** |
| **Stomach** | **0.24 ± 0.05** | **0.36 ± 0.06** | **0.40 ± 0.05** | **0.43 ± 0.04** | **0.41 ± 0.03** |
| **Liver** | **0.27 ± 0.06** | **0.37 ± 0.07** | **0.41 ± 0.06** | **0.49 ± 0.01** | **0.41 ± 0.09** |
| **Spleen** | **0.31 ± 0.08** | **0.50 ± 0.11** | **0.51 ± 0.09** | **0.48 ± 0.11** | **0.47 ± 0.04** |
| **Pancreas** | **0.98 ± 0.25** | **1.76 ± 0.16** | **2.55 ± 0.31** | **2.28 ± 0.76** | **2.24 ± 0.72** |
| **Small intestine** | **0.23 ± 0.11** | **0.52 ± 0.26** | **0.65 ± 0.04** | **0.43 ± 0.11** | **0.41 ± 0.09** |
| **Large intestine** | **0.21 ± 0.01** | **0.28 ± 0.05** | **0.42 ± 0.06** | **0.46 ± 0.04** | **0.38 ± 0.09** |
| **Kidney** | **0.33 ± 0.01** | **0.34 ± 0.06** | **0.41 ± 0.08** | **0.42 ± 0.07** | **0.41 ± 0.09** |
| **Muscle** | **0.19 ± 0.03** | **0.28 ± 0.01** | **0.36 ± 0.04** | **0.44 ± 0.10** | **0.37 ± 0.10** |
| **Left brain** | **0.08 ± 0.01** | **0.25 ± 0.05** | **0.40 ± 0.06** | **0.48 ± 0.14** | **0.43 ± 0.09** |
| **Right brain** | **0.07 ± 0.02** | **0.21 ± 0.03** | **0.34 ± 0.03** | **0.48 ± 0.08** | **0.34 ± 0.04** |
| **Tumor** | **0.12 ± 0.02** | **0.43 ± 0.10** | **1.26 ± 0.22** | **1.49 ± 0.40** | **1.02 ± 0.16** |

### Example 6: Positron emission tomography (PET) of tumor-bearing rat with L-[¹⁸F]FET

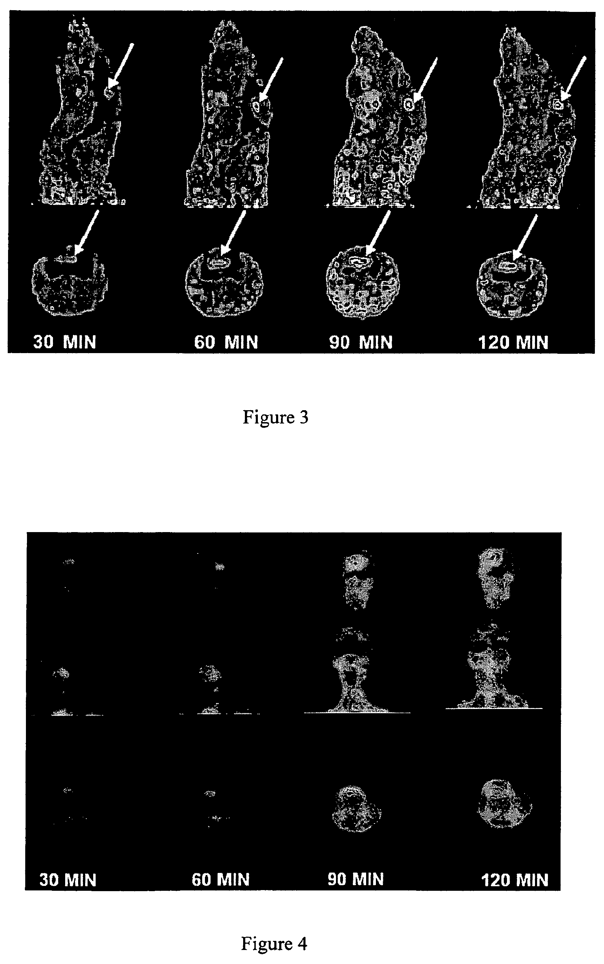

(1) Positron emission tomography (PET) studies of tumor-bearing rat (Fischer 344) with L-[¹⁸F]FET will be carry out after 11~12 days of brain tumor (F98 glioma) implantation.
(2) Producing processes of L-[¹⁸F]FET as described above, and estimates its administrating amount of agent according to the level of radioactivity.
(3) At the very beginning, inject 400 µCi of L-[¹⁸F]FET into tail vein. Temporarily anesthetize rats with ether at time of 0.5, 1, 1.5 and 2 hours. Fix rat on transparent acrylic board and place on PET table after securing stretched limbs on the board. Continuously anesthetize rats with isoflurane gas. Align implant portion of head with PET machine as to assure the fixation of original imaging position.
(4) Adjust background value by blank scan before PET imaging.
(5) Use Ga-68 radioactive source proceed transmission scan for adjust tissue attenuation.
(6) Commence PET and microPET imaging. Perform image processing after reconstruction of acquired image data; the coronal and cross section scans show in Figure 3 and 9. Rat injected L-[¹⁸F]FET has significant radioactivity accumulation in brain tumor and reach maximum uptake after 90 minutes of injection. In contrast to tumor site, normal tissue have lower radioactivity accumulation of L-[¹⁸F]FET because of lower amino acid requirement. PET imaging of L-[¹⁸F]FET shows the definite location of tumor; this finding is totally consistent with the result of biological distribution, i.e., the accumulation of L-[¹⁸F]FET and tumor-to-normal brain ratio of brain tumor is much higher than that of normal organs except pancreas.

## Claims

1. A method for produce a precursor of ¹⁸F-labeled *O*-(2-[¹⁸F]fluoroethyl)-L-Tyrosine, which has the following chemical structure: 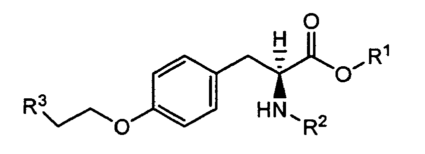 wherein R¹ is a protective group for the carboxyl functional group and represents an arylalkyl group;
R² is a protective group for the amino group and represents a carboxyl group; and
R³ acts as a leaving group and represents ap-tosyloxy, methane sulfonyloxy or trifluoromethane sulfonyloxy or bromine.

2. A method to produce *O*-(2-[¹⁸F]fluoroethyl)-L-Tyrosine utilizes the precursor according to claim 1as an original material, and by the reaction Kryptofix 2.2.2 as a catalyst with ¹⁸F-radioisotope.

3. A method to produce *O*-(2-[¹⁸F]fluoroethyl)-L-Tyrosine utilizes the precursor according to claim 1 as an original material, and by the reaction TBAHCO₃ as a catalyst with ¹⁸F-radioisotope.

4. The method according to claim 2, wherein the production of *O*-(2-[¹⁸F]fluoroethyl)-L-Tyrosine is by Kryptofix 2.2.2 as a catalyst and utilizes Silica gel column for purification.

5. The method according to claim 2, wherein the production of *O*-(2-[¹⁸F]fluoroethyl)-L-Tyrosine is by the reaction TBAHCO₃ as a catalyst with ¹⁸F-radioisotope.

6. An injectable solution produce by *O*-(2-[¹⁸F]fluoroethyl)-L-Tyrosine according to claim 2, which can be used as a radiopharmaceuticals for diagnosis and treatment efficacy follow-up of tumor.

7. An injectable solution produce by *O*-(2-[¹⁸F]fluoroethyl)-L-Tyrosine according to claim 3, which can be used as a radiopharmaceuticals for diagnosis and treatment efficacy follow-up of tumor.

## Amended claims

### Amended claims in accordance with Rule 86(2) EPC.

**1.** A method to produce O-(2-[18F]fluoroethyl)-L-Tyrosine utilizes the precursor of formula 1 as an original material, and by the reaction Kryptofix 2.2.2 as a catalyst with 18F-radioisotope; 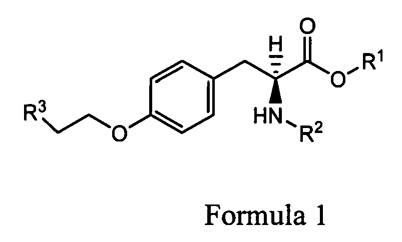 wherein R1 is a protective group for the carboxyl functional group and represents an arylalkyl group;
R2 is a protective group for the amino group and represents a carboxyl group; and
R3 acts as a leaving group and represents a p-tosyloxy, methane sulfonyloxy or trifluoromethane sulfonyloxy or bromine.

**2.** The method according to claim 1, wherein the production of O-(2-[18F]fluoroethyl)-L-Tyrosine is by Kryptofix 2.2.2 as a catalyst and utilizes Silica gel column for purification.
